# EUROPEAN PATENT SPECIFICATION

(11) **EP 2 156 751 B1**
(45) Date of publication and mention of the grant of the patent: **03.10.2018**
(21) Application number: 08751758.7
(22) Date of filing: 19.05.2008
(51) Int. Cl.: A23L 5/00, A23L 33/125, A23L 2/60, A61K 31/7004, A23L 29/30, A23L 27/30

(54) **NOVEL SWEETENER HAVING SUGAR-LIKE TASTE AND PRODUCTION METHOD AND USE OF THE SAME**
NEUER SÜSSSTOFF MIT ZUCKERARTIGEM GESCHMACK, VERFAHREN ZU SEINER HERSTELLUNG UND SEINE VERWENDUNG
EDULCORANT INÉDIT AU GOÛT DE SUCRE, SON PROCÉDÉ DE FABRICATION ET SON UTILISATION

(30) Priority: 18.05.2007 JP 2007133291
(43) Date of publication of application: 24.02.2010
(73) Proprietor: Matsutani Chemical Industry Co., Ltd., Itami-shi, Hyogo 664-8508 (JP); National University Corporation Kagawa University, Takamatsu-shi, Kagawa 760-8521 (JP); Rare Sugar Production Technical Research Laboratories, LLC, Mikicho Kita-gun Kagawa 761-0615 (JP); Kabushiki Kaisha Hayashibara Seibutsu Kagaku Kenkyujo, Okayama-shi Okayama 700-0907 (JP)
(72) Inventor: OKUMA, Kazuhiro, Itami-shi Hyogo 664-8508 (JP); YAMADA, Koji, Itami-shi Hyogo 664-8508 (JP); TSUKUDA, Koji, Itami-shi Hyogo 664-8508 (JP); IIDA, Tetsuo, Itami-shi Hyogo 664-8508 (JP); OGA, Hiroshi, Itami-shi Hyogo 664-8508 (JP); IZUMORI, Ken, Kita-gun Kagawa 761-0793 (JP); TSUJISAKA, Yoshio, Kita-gun Kagawa 761-0615 (JP); SHIMONISHI, Tsuyoshi, Kita-gun Kagawa 761-0615 (JP); YAMADA, Takako, Itami-shi Hyogo 664-8508 (JP); OKAMOTO, Iwao, Okayama-shi Okayama 700-0907 (JP)
(74) Representative: TBK
(86) International application number: PCT/JP2008/001240
(87) International publication number: WO 2008/142860

(56) References cited:
- EP-A1- 1 864 669
- EP-A1- 2 098 227
- EP-A1- 2 111 865
- WO-A1-2006/101118
- WO-A1-2008/056453
- CA-C- 1 292 988
- JP-A- 2001 011 090
- JP-A- 2001 011 090
- JP-A- 2007 051 137
- US-A- 3 285 776
- MATSUO T ET AL: "Effects of dietary D-psicose on diurnal variation in plasma glucose and insulin concentrations of rats", BIOSCIENCE BIOTECHNOLOGY BIOCHEMISTRY, JAPAN SOCIETY FOR BIOSCIENCE, BIOTECHNOLOGY, AND AGROCHEMISTRY, TOKYO, JAPAN, vol. 70, no. 9, 1 September 2006 (2006-09-01), pages 2081-2085, XP003022571, ISSN: 0916-8451, DOI: 10.1271/BBB.60036
- MATSUO T ET AL: "Less Body Fat Accumulation with D-Psicose Diet versus D-Fructose Diet", JOURNAL OF CLINICAL BIOCHEMISTRY AND NUTRITION, TOKYO, JP, vol. 30, 1 January 2001 (2001-01-01), pages 55-65, XP003003027, ISSN: 0912-0009
- KOSAKAI T. ET AL.: 'D-tagatose 3-epimerase Hanno ni Okeru D-fructose/D-psicose Kongo Toeki no Kesshoka' ABSTRACTS OF THE ANNUAL MEETING OF THE SOCIETY FOR BIOTECHNOLOGY JAPAN 2004, page 77 + ABSTR. NO. 1PD16-5, XP008118276
- MATSUO T. ET AL.: 'D-psicose is a rare sugar that provides no energy to growing rats' J. NUTR. SCI. VITAMINOL. (TOKYO) vol. 48, no. 1, 2002, pages 77 - 80, XP002973184
- NAZUMORI K. ET AL.: 'Kishoto D-psicose no Seisan Oyobi Shokuhin Sozai to Shiteno Kanosei' THE FOOD INDUSTRY vol. 48, no. 17, 2005, pages 46 - 55, XP008118278
- ITOH H ET AL: "Preparation of D-psicose from D-fructose by immobilized D-tagatose 3-epimerase", JOURNAL OF FERMENTATION AND BIOENGINEERING, SOCIETY OF FERMENTATION TECHNOLOGY, JP, vol. 80, no. 1, 1 January 1995 (1995-01-01), pages 101-103, XP003003992, ISSN: 0922-338X, DOI: 10.1016/0922-338X(95)98186-O

## Description

### Technical Field

The present invention relates to a composition (sweetener) including D-glucose, D-fructose, and D-psicose, which is obtained by the action of an isomerase and an epimerase on glucose liquid sugar, and uses of the same.

### Background Art

Isomerized sugar is obtained by saccharifying starch to give a saccharified solution, followed by treatment with glucose isomerase. Because of its low production cost, isomerized sugar has been widely used to add sweetness to soft drinks and other beverages. In the United States, more than eight million tons of isomerized sugar is consumed. In a typical method, starch is enzymatically hydrolyzed to dextrin, and the obtained dextrin is further hydrolyzed by another enzyme, thereby giving a glucose solution, i.e., saccharified solution. Such a saccharified solution contains, in addition to glucose, a small amount of oligosaccharide. Isomerization of glucose by glucose isomerase to fructose is an equilibrium reaction, and the ratio between glucose and fructose in isomerized sugar is usually about 58:42. Further, in order to compensate for low sweetness, refined fructose is occasionally added thereto. In such a case, the final ratio between glucose and fructose is usually about 45:55. Isomerized sugar is widely used for its economic benefits as mentioned above; however, in advanced nations including Japan, the United States, etc., isomerized sugar has been suspected to cause hyperglycemia and overweight (obesity) ("metabolic syndrome") (Nonpatent Document 1). As compared with glucose, metabolism of fructose in the liver is more likely to accelerate lipogenesis, inducing hyperlipidemia and obesity. For this reason, attention has been focused on the relation between the intake of fructose contained in fructose/glucose liquid sugar and the increase in the number of obese people (lifestyle-related disease).

Isomerized sugar is different from commonly used sucrose in the degree and quality of sweetness. Although attempts have been made, for example, to change the ratio between glucose to fructose therein to resemble the degree and quality of sweetness of sucrose, satisfactory results have not yet been obtained.

Meanwhile, D-psicose is a kind of rare sugar, and is produced from D-fructose by the action of D-ketohexose 3-epimerase (Patent Document 1) thereon at a yield of 20 to 25%. Further, it has been reported that in the case of using D-psicose 3-epimerase (Nonpatent Document 2), D-psicose is produced at a yield of 40%, while in the case of using boric acid together, D-psicose is produced at 62%. Where refined D-fructose is first produced and D-psicose is then produced, because they are produced in different processes, restrictions are imposed regarding the costs of raw materials, transportation, reaction, plant operation, etc. The reality is that industrial mass production of D-psicose is extremely difficult.

The characteristics of D-psicose as a material for preventing lifestyle-related diseases have been revealed, including its noncaloric nature (Nonpatent Document 3), suppressing effect on postprandial blood glucose level (Nonpatent Document 4), antiobesity effect (Nonpatent Document 5), and the like.

Further, the sweetness of D-psicose is about 70% of that of sucrose, and therefore, when used singly as a sweetener, the degree and quality of sweetness thereof will differ from the case of sucrose.

Patent Document 1: JP-A-6-125776
Nonpatent Document 1: Am. J. Clin. Nutr. 2004, 79, 774-9
Nonpatent Document 2: The 3^{rd} Symposium of International Society of Rare Sugars
Nonpatent Document 3: J. Nutr. Sci. Vitaminol. 48, 77-80
Nonpatent Document 4: J. Nutr. Sci. Vitaminol. 59, 191-121
Nonpatent Document 5: J. Clin. Biochem. Nutr., 30, 55-65

JP 2001 011090 A describes a method for preparing a crystalline glucides complex including D-psicose and D-fructose.

### Disclosure of the Invention

### Problems that the Invention is to Solve

An object of the invention is to solve the primary problem of fructose/glucose liquid sugar that has been widely used in the food industry as a sweetener, i.e., the problem that the degree and quality of sweetness thereof is different from that of sucrose, and thus provide the uses of a sweetener closely similar to sucrose in sweetness and taste, which is obtained by improvement of the taste of D-psicose.

Another object of the invention is to solve the secondary problem of fructose/glucose liquid sugar that has been widely used in the food industry as a sweetener, i.e., the problem that it causes lifestyle-related diseases, such as obesity, etc., and thus provide the use of a sweetener closely similar to sucrose in sweetness and taste, which is obtained by improvement of the taste of D-psicose.

### Means for Solving the Problems

The present inventors conducted extensive research to solve the above problems. As a result, they found that a novel use of sweetener as defined in claims 1 and 2 including D-glucose, D-fructose, and D-psicose at a specific ratio is similar to sucrose in the degree and quality of sweetness and is also effective in preventing lifestyle-related diseases, such as obesity induction by isomerized sugar. The invention was thus accomplished.

The invention provides sweeteners as defined in claims 1 and 2.

(5) The sweetener for the uses as defined in the claims having the sweetness and taste of sucrose is obtainable by the action of an isomerase-epimerase mixed enzyme on glucose liquid sugar.

The invention also provides the non-therapeutic use of a sweetener having the sweetness and taste of sucrose as defined in claims 3 and 4 for suppressing an increase in body weight or an increase in body fat accumulation.

### Advantage of the Invention

The invention solves the primary problem of fructose/glucose Liquid sugar that has been widely used in the food industry as a sweetener, i.e., the problem that the degree and quality of sweetness thereof are different from those of sucrose, and thus provides a sweetener closely similar to sucrose in sweetness and taste, which is obtained by improvement of the taste of D-psicose.

The invention also solves the secondary problem of fructose/glucose liquid sugar that has been widely used in the food industry as a sweetener, i.e., the problem that it causes lifestyle-related diseases, such as obesity, etc., and thus provides a sweetener closely similar to sucrose in sweetness and taste, which is obtained by improvement of the taste of D-psicose.

### Brief Description of the Drawings

FIG. 1 explains the production of D-psicose from isomerized sugar.
FIG. 2 shows sweetness curves.
FIG. 3 shows changes in body weight in the experimental method (1).
FIG. 4 shows differences in body fat in the experimental method (1).
FIG. 5 shows differences in ketone body in the experimental method (1).
FIG. 6 shows changes in body weight in the experimental method (2).
FIG. 7 shows differences in body fat in the experimental method (2).
FIG. 8 shows differences in ketone body in the experimental method (2).

### Best Mode of Carrying Out the Invention

A specific embodiment of the sweetener of the invention is the use of a sweetener having D-fructose in an amount of 30 to 80 parts and D-glucose and D-psicose in a total amount of about 70 to about 20 parts by weight. Based on 100 parts by weight of the totel amount of the D-glucose and the D-psicose, the amount of the D-psicose is not less than 5 parts by weight, preferably not less than 10 parts by weight. Such a sweetener is close to sucrose in sweetness and taste, and prevents lifestyle-related diseases, such as obesity.

In overview, the sweetener of the invention is a mixture of its components, i.e., D-fructose, D-glucose, and D-psicose, and obtained by mixing the components. Fructose and glucose are monosaccharides that generally exist in nature, and are obtainable by isolation from the natural environment. Fructose can also be obtained by isolation from fructose/glucose liquid sugar or the like produced by treating glucose with glucose isomerase. Glucose is produced by hydrolysis of starch. Psicose is a kind of rare sugar that barely exists in nature, and is obtainable by treating fructose with a ketohexose 3-epimerase.

The overview of the method for producing a sweetener of the invention is as follows. The sweetener is obtainable by mixing the structural components. In addition, it may also be obtained by adding D-psicose to D-fructose/D-glucose liquid sugar that is produced by the action of glucose isomerase on D-glucose, a raw material, or may alternatively be obtained by the further action of D-ketose 3-epimerase on the thus-obtained D-fructose/D-glucose liquid sugar to thereby partially convert D-fructose to D-psicose. Further, it is also possible to allow glucose isomerase and D-ketose 3-epimerase to simultaneously act on a D-glucose solution, thereby producing the novel sweetener including D-glucose, D-fructose, and D-psicose at the above specific ratio in one process. In terms of cost, advantageous methods are those in which the process of glucose isomerase treatment (isomerization) of D-glucose and the process of D-ketose 3-epimerase treatment are directly connected, as well as those that use a mixed enzyme system of glucose isomerase and D-ketose 3-epimerase to produce the intended sweetener from D-glucose in one operation.

The novel sweetener of the invention has a ratio of D-fructose to the (total of D-glucose and D-psicose) of 80 to 30 parts by weight: 20 to 70 parts by weight. When the amount of D-fructose exceeds 80 parts by weight, sweetness increases, while the richness of taste decreases.

When D-fructose is less than 20 parts by weight, the resulting sweetness is likely to be unsatisfactory. When the amount of D-psicose is not less than 5 parts by weight based on 100 parts by weight of the total amount of D-glucose and D-psicose, the resulting sweetener has the sweetness and taste of sucrose. When the amount is not less than 10 parts by weight, obesity-preventing effects are provided.

Hereinafter, the method for producing the sweetener of the invention will be explained in detail. Where D-fructose/D-glucose liquid sugar, D-glucose, or starch is used as a starting material, the sweetener of the invention can be produced by various methods. For example, (1) D-fructose/D-glucose liquid sugar is produced from starch or D-glucose, and ketose 3-epimerase acts thereon to produce D-psicose. (2) Glucose isomerase and ketose 3-epimerase act as a mixed enzyme on degraded D-glucose liquid sugar that is obtained by degradation of starch. (I) Method using a continuous plant (degraded fructose/glucose liquid sugar ⇒ ketose 3-epimerase).

### (1) Preparation of degraded D-fructose/D-glucose liquid sugar

Degraded D-fructose/D-glucose liquid sugar is produced in the usual method using starch such as corn, potato, sweet potato as a raw material, by applying enzymes such as immobilized or a batch of alpha amylase, glucoamylase, glucose isomerase. The kinds of starch raw materials and enzymes used are not limited to the above examples. If necessary, a D-glucose solution may be produced by acidolysis, and isomerization using alkali is also possible.

### (2) Production of D-psicose from D-fructose/D-glucose liquid sugar solution

The degraded isomerized sugar solution produced is continuously subjected to the action of an epimerase, thereby giving a mixed sugar solution of D-glucose, D-fructose, and D-psicose.

### (II) Method using a mixed enzyme (degraded glucose liquid sugar ⇒ glucose isomerase + ketose 3-epimerase)

An immobilized enzyme containing an isomerase and an epimerase is packed into a suitable column, and degraded D-glucose liquid sugar is continuously poured thereinto. The reaction mixture is then fractionated. In this case, the starting material may be starch in place of D-glucose, and a mixed enzyme system further containing alpha amylase and glucoamylase may also be used.

In the production of the sweetener of the invention, the glucose isomerase is an enzyme that acts on D-glucose to partially convert it to D-fructose, and may be a refined enzyme. Alternatively, microorganisms capable of producing such an enzyme may also be used. Further, the ketohexose 3-epimerase is an enzyme that isomerizes OH in the 3-position of a ketohexose such as D-fructose. Known examples thereof are D-tagatose 3-epimerase and D-psicose 3-epimerase (References: Patent No. 3333969, D-ketohexose 3-epimerase obtainable from bacteria of genus Pseudomonas, and Nonpatent Document 2). It may also be a refined enzyme, an immobilized enzyme obtained by immobilizing microorganisms capable of producing such an enzyme, and immobilized microorganisms.

When tagatose 3-epimerase as the ketohexose 3-epimerase acts on ordinary isomerized sugar including, as constituent saccharides, 42 parts by weight of D-fructose and 58 parts by weight of D-glucose, this produces 58 parts by weight of D-glucose, 34 parts by weight of D-fructose, and 8 parts by weight of D-psicose. However, when a mixed enzyme of glucose isomerase and tagatose 3-epimerase acted on D-glucose, unexpectedly, a mixture of 41 parts by weight of D-glucose, 48 parts by weight of D-fructose, and 11 parts by weight of D-psicose was obtained. As compared with conventional D-fructose/D-glucose liquid sugar, such mixtures were closer to sucrose in the degree and quality of sweetness. In addition, the sweetness and taste of such a mixture can be adjusted by further adding D-fructose or D-glucose thereto.

If desired, the thus-obtained novel sweetener can be used in combination with other sweeteners, such as sucrose, sugar alcohol, aspartame, and stevia. Further, in order to enrich the taste, water-soluble dietary fibers with low sweetness (polydextrose, inulin, indigestible dextrin, etc.) may be suitably added thereto.

In the sweetener of the invention, the amount of D-fructose is 30 parts by weight to 80 parts by weight, and the (total amount of D-glucose and D-psicose) is about 70 parts by weight to about 20 parts by weight. However, in an application taking advantage of the degree and quality of sweetness thereof, the content is not limited, and may be suitably adjusted according to the intended function and usage, the used amount, and the like.

The sweetener of the invention contains D-psicose and is effective in preventing obesity.

Further, for the prevention of lifestyle-related diseases, it may also be used in combination with additional active ingredients.

The sweetener of the invention can be applied to any products that need sweetness, examples thereof including foods, foods for health, foods for patients, food materials, food materials for health, food materials for patients, food additives, food additives for health, food additives for patients, beverages, beverages for health, beverages for patients, potable water, potable water for health, potable water for patients, drugs, drug raw materials, feeds, and feeds for diseased domestic animals and/or diseased animals.

When applied to foods, the sweetener of the invention may be used as it is, or may also be used in the form of a dilution with water or the like, a suspension in oil or the like, an emulsion, a mixture with a carrier generally used in the food industry, or the like. When applied to beverages, it may be in the form of a nonalcoholic beverage or an alcoholic beverage. Examples of nonalcoholic beverages include carbonated beverages, non-carbonated beverages such as fruit juices and nectar beverages, soft drinks, sports drinks, tea, coffee, and cocoa. Examples of alcoholic beverages include beer, low-malt beer, third beer, sake, plum liquor, wine, champagne, liqueurs, cocktails, and medicinal alcoholic beverages.

When the composition (sweetener) of the invention is used as a food material or a food additive for alleviating abnormal carbohydrate metabolism and/or abnormal lipid metabolism, it may be in the form of a solid preparation such as a tablet, a capsule, or a powder or granules to be dissolved in beverages, etc.; a semisolid preparation such as jelly; a liquid such as potable water; a high-concentration solution to be diluted before use; or the like.

Further, the composition (sweetener) of the invention may be suitably added to foods to give healthy diets or foods for patient, which are used for alleviating abnormal carbohydrate metabolism and/or abnormal lipid metabolism. As optional components, vitamins, carbohydrates, coloring agents, flavoring agents, and the like, which are ordinary food additives may be suitably added. The food may be taken in an arbitrary liquid or solid form. It may be encapsulated in gelatin or the like, and taken as a soft capsule. Such a capsule is formed using a gelatin shell, for example, which is prepared by adding water to the raw material gelatin to dissolve, and adding a plasticizer (glycerin, D-sorbitol, etc.) thereto.

The sweetener of the invention can be used for the same purposes as in the case of sucrose, and may also be used for cooking or in tea, coffee, seasonings (*mirin* (sweet rice wine for cooking), etc.), and the like.

Specific examples of food and drink include western confectioneries (puddings, jellies, gumdrops, candies, drops, caramels, chewing gums, chocolates, pastries, butter creams, custard, cream puffs, pancakes, breads, potato chips, fried potatoes, popcorn, biscuits, crackers, pies, sponge cakes, waffles, cakes, doughnuts, biscuits, cookies, rice crackers, *okoshi* (millet-and-rice cakes), steamed buns, candies, etc.), dried noodles (macaroni, pasta), egg products (mayonnaise, fresh cream), beverages (functional beverages, lactic acid beverages, lactic acid bacteria beverages, thick lactic beverages, fruit juice beverages, beverages containing no fruit juice, fruit juice beverages containing pulp, clear carbonated beverages, carbonated beverages containing fruit juice, fruit-colored carbonated beverages), nonessential grocery items (green tea, black tea, instant coffee, cocoa, canned coffee beverages), dairy products (ice cream, yogurt, milk for coffee, butter, butter sauce, cheese, fermented milk, processed milk), pastes (marmalade, jam, flower paste, peanut paste, fruit paste, fruits preserved in syrup), meat products (ham, sausage, bacon, dry sausage, beef jerky, lard), seafood products (fish ham, fish sausage, boiled fish paste, *chikuwa* (tube-shaped fish sausage), *hanpen* (cake of ground fish), dried fishes, dried bonito, dried mackerel, dried sardine, sea urchin eggs, *shiokara* (salted and fermented fish guts) of squid, dried cuttlefish, dried fishes seasoned with *mirin,* dried shellfishes, smoked salmon or the like), *tsukudani* (foods boiled down in soy) (small fishes, shellfishes, wild vegetables, mushrooms, kelp), curries (instant curry, retort curry, canned curry), seasonings (bean paste, powdered bean paste, soy sauce, powdered soy sauce, *moromi* (unrefined sake or soy sauce), fish sauce, sauce, catsup, oyster sauce, bouillon cube, barbecue sauce, curry roux, stew base, powdered soup, powdered broth, paste, instant soup, *furikake* (dry condiment to be sprinkled over rice), dressing, salad oil), fried products (deep-fried tofu, deep-fried tofu confectioneries, instant Chinese noodles), soybean milk, margarine, and shortening.

The above food and drink can be prepared by mixing the composition with ordinary food materials in the usual manner. The amount of the composition to be added to the above food and drink varies depending on the kind of food, and is not limited. Usually, an amount of 0.1 to 50 wt% is preferable.

The above food and drink can be used also as functional foods, dietary supplements, or health foods. The form thereof is not limited. Examples of food products are highly nutritious proteins with a good amino acid balance, such as milk protein, soybean protein, and ovalbumin; decomposition products thereof; albumen oligopeptide; soybean hydrolysates; mixtures of simple amino acids; and the like. They can be used in the usual manner, and may also be used in the form of soft capsules, tablets, etc.

Examples of supplements and functional foods include preparations containing saccharides, fats, trace elements, vitamins, emulsifiers, flavoring agents, etc., in the form of liquid diets, defined formula diets, elementary diets, health drinks, capsules, enteral nutrients, or the like. Among the above-mentioned food products, beverages such as an isotonic drink, or a nutrition supplement drink, for example, may further contain, in order to improve the nutritional balance and flavor thereof, nutritional additives such as amino acids, vitamins, and minerals, sweeteners, spices, flavoring agents, coloring agents, and the like.

The composition of the invention is applicable to feeds for domestic animals, domestic fowls, and companion animals. For example, the composition can be added to dried dog foods, dried cat foods, wet dog foods, wet cat foods, semi-moist dog foods, feeds for poultry farming, and feeds for livestock including cows, pigs, etc. The feed itself can be prepared in the usual manner.

Such therapeutic agents and prophylactic agents can be used for not only humans but also other animals, including domestic mammals such as cows, horses, pigs, and sheep, domestic poultry including chickens, quails, and ostriches, companion animals such as reptiles, birds, and small mammals, farmed fishes, and the like, for example.

A drug aimed at developing physiological functions, while taking advantage of the sweetness of the sweetener of the invention, which is used for the purpose of alleviating abnormal carbohydrate metabolism and/or abnormal lipid metabolism and also obesity, may be used singly. Alternatively, suitable additives such as general excipients, stabilizers, preservatives, binders, and disintegrators, may be added thereto to formulate the same into a suitable pharmaceutical form such as a solution, granules, subtle granules, a powder, a tablet, a capsule, a pill, a dusting powder, or a spray. Such a preparation may be administered orally or nasally.

For the preparation of the composition of the invention as a drug, it is possible to use an organic or inorganic solid, semisolid, or liquid carrier, resolvent, or diluent for medical use suitable for oral administration or nasal administration. Water, gelatin, lactose, starch, magnesium stearate, talc, animal/vegetable oil, benzyl alcohol, gum, polyalkylene glycol, petroleum resin, coconut oil, lanolin, and other carriers for medical use can all be used as carriers for a drug containing the composition of the invention. In addition, a stabilizer, a humectant, an emulsifier, or a salt for changing the osmotic pressure or maintaining the appropriate pH of the added agents may also be used as an adjuvant agent.

The invention is also applicable to toothpaste or the like as a sweetener.

Further, recently, in the preparation of cosmetics and the like, soluble films have been used. For example, for the purpose of providing refreshment or preventing halitosis, an edible soluble film has been used as a flavored film or the like containing a flavoring agent or the like. As a packaging material for foods, drugs, and the like, and also as a carrier for supporting active ingredients of foods, drugs, and the like, a soluble film having excellent solubility and film characteristics and suitable for such usages has been proposed (JP-A-2007-091696). In this manner, the sweetener having the sweetness and taste of sucrose of the invention can be applied to drugs, quasi drugs, or cosmetics.

Hereinafter, the invention is explained in further detail with reference to the examples, but the scope of the invention is not limited thereto.

### Example 1

### Preparation of sugar solution containing D-fructose, D-glucose, and D-psicose

### (1) Method using a continuous plant

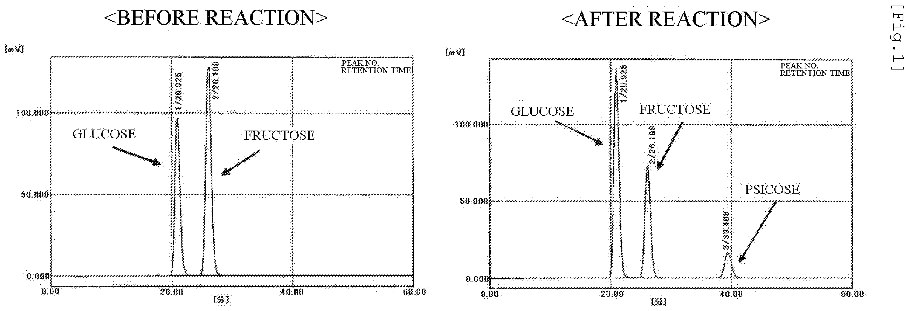

Water and α-amylase (from Bacillus lichenifomis) were added to starch, and degradation was caused at approximately 95°C. After the completion of liquefaction, the mixture was cooled to 55°C. Glucoamylase (from Chalara paradoxa) was added to cause further degradation, and a glucose solution was thus produced. The obtained glucose liquid sugar was poured into a column with immobilized glucose isomerase (from Bacillus coagulans) at 60°C, and isomerized sugar was thus produced. As a D-ketohexose 3-epimerase, D-tagatose 3-epimerase (from genus Pseudomonas) was used. Preparation of the enzyme and measurement of the enzyme activity were performed according to a patent document (JP-A-2001-11090), etc. That is, a 50 mM Tris-HCl buffer containing an isomerized sugar solution (10% (w/v), containing D-fructose) was poured into a column packed with Chitopearl beads BCW2503 (manufactured by FUJI SPINNING) with immobilized D-tagatose 3-epimerase, while maintaining the temperature at 45°C. About 1 l of the reaction mixture eluted from the column was recovered, and then, in the usual manner, concentrated, treated with activated carbon and a membrane, and desalted using a cation exchange resin and an anion exchange resin. The obtained sugar solution was analyzed by HPLC (detection: RI, column: MITSUBISHI KASEI, MCI GEL CK 08EC) . The analysis showed that the sugar solution had, approximately, 58 parts by weight of D-glucose, 34 parts by weight of D-fructose, and 8 parts by weight of D-psicose (FIG. 1) .

### (2) Method using a mixed enzyme

Glucose isomerase (11.4 U) (from Bacillus coagulans), D-tagatose 3-epimerase (6.2 U) (from genus Pseudomonas), and 50 mM Tris-HCl buffer containing 1% (w/v) glucose liquid sugar were allowed to react while maintaining the temperature at 30°C. The mixture was purified as in (1), and analyzed by HPLC.

The analysis showed that after reacted for 2 hours, the sugar solution had, approximately, 41 parts by weight of D-glucose, 48 parts by weight of D-fructose, and 11 parts by weight of D-psicose. From the fact that in the case where the reaction time is 1 hour, the solution has 43 parts by weight of D-glucose, 48 parts by weight of D-fructose, and 6 parts by weight of D-psicose, it was accordingly shown that the reaction ratio can be controlled to some extent by changing the reaction time.

As compared with the case where a mixed sugar solution is produced from isomerized sugar, this method results in a better balance between D-fructose and D-psicose and also a higher yield.

(3) Method for preparing high-psicose-content sugar solution To 10 % (W/V) glucose liquid sugar was added a mixed enzyme containing 50 g of immobilized glucose isomerase (from Bacillus coagulans; manufactured by NOVO INDUSTRY) per kg of glucose solid content and 2000 u of D-tagatose 3-epimerase (from genus Pseudomonas) per kg of glucose solid content. The mixture was allowed to react at 65°C and pH 7.5 for 4 hours or 20 hours. The reaction sugar solution was treated with a membrane and desalted in the usual manner, and then analyzed by HPLC (detection: RI, column: MITSUBISHI KASEI, MCI GEL CK 08EC). After reacted for 4 hours, the sugar solution had 54 parts by weight of D-glucose, 36 parts by weight of D-fructose, and 10 parts by weight of D-psicose, while after reacted for 20 hours, the sugar solution had 41 parts by weight of D-glucose, 42 parts by weight of D-fructose, and 17 parts by weight of D-psicose. This therefore shows that by the method (2) using a mixed enzyme, a sugar solution having a psicose content as high as 10% or more can be easily produced.

### Example 2

### Sensory testing of sugar solution containing D-fructose, D-glucose, and D-psicose

Several 10% (w/v) sugar solutions having D-fructose and/or D-glucose and/or D-psicose at various ratios were prepared, and subjected to taste testing by nine panelists. For evaluation of sweetness, the panelists were asked to select "light", "same", or "heavy" in comparison with a sugar solution having the same degree of sweetness as that of sucrose and also with sucrose. The number of panelists who selected each option was determined. The results of the sensory testing are shown in Table 1. The panelists were nine men and women in their twenties to thirties having a good sense of taste.

**[Table 1]**

| Results of sensory testing of sugar solution | | | | | | | | | | | | | | | |
|---|---|---|---|---|---|---|---|---|---|---|---|---|---|---|---|
| | | A | B | C | D | E | F | G | H | I | J | K | L | M | N |
| Proportion of sugars (%) | Fructose | 0 | 10 | 20 | 30 | 34 | 40 | 50 | 55 | 55 | 60 | 70 | 80 | 90 | 55 |
| | Glucose: psicose (1:1) | 100 | 90 | 80 | 70 | | 60 | 50 | | | 40 | 30 | 20 | 10 | |
| | Glucose | | | | | 60 | | | 15 | 35 | | | | | 45 |
| | Psicose | | | | | 6 | | | 30 | 10 | | | | | |
| Degree of sweetness | | 0 | 1 | 0 | 4 | 5 | 6 | 7 | 7 | 5 | 5 | 4 | 5 | 2 | 3 |
| Quality of sweetness | Light | 8 | 9 | 7 | 3 | 3 | 3 | 2 | 4 | 2 | 3 | 3 | 1 | 3 | 5 |
| | Same | 1 | 0 | 2 | 6 | 6 | 5 | 7 | 5 | 5 | 3 | 5 | 4 | 1 | 3 |
| | Heavy | 0 | 0 | 0 | 0 | 0 | 1 | 0 | 0 | 2 | 3 | 1 | 4 | 5 | 1 |

The degree and quality of sweetness of isomerized sugar changes depending on the ratio between D-fructose content and D-glucose content. In sensory testing on isomerized sugar (55 parts by weight of fructose:45 parts by weight of glucose), many panelists commented that they felt lighter sweetness as compared with sucrose. Also in the above results, only three panelists perceived similarity in the degree of sweetness, and only three panelists perceived similarity in the quality of sweetness. This therefore indicates that the taste and sweetness thereof are distinct from those of sucrose. Meanwhile, the results of the sensory testing showed that a combination that is similar to sucrose in sweetness and/or taste is a mixture of sugars containing fructose in an amount of 30 parts by weight to 80 parts by weight and D-glucose and D-psicose in an amount of (70 parts by weight to 20 parts by weight). Further, as a result for the sample E, a glucose:psicose ratio of about 9:1 provided the degree and quality of sweetness similar to that of sucrose. This therefore shows that based on 100 parts by weight of the total amount of glucose and psicose, when the amount of psicose is not less than 10 parts by weight, a desired degree and quality of sweetness is obtained. It is known that, generally, as compared with sweeteners formed of a single component, mixed sugars have a wider peak of sweetness and exhibit milder sweetness characteristics.

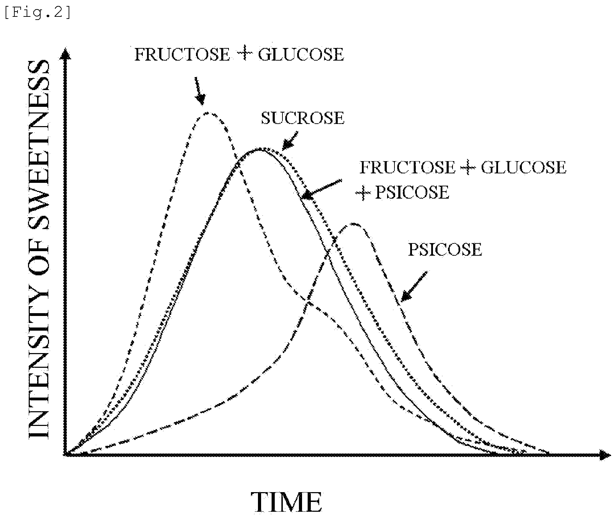

Meanwhile, D-fructose has a sharp, refreshing taste, while D-psicose has such a characteristic that the sweetness is slowly perceived. It thus appears that the addition of D-psicose changed the sweetness curve from the isomerized-sugar curve (fructose + glucose) to the mixed sugar curve (fructose + glucose + psicose) as shown in FIG. 2, whereby the resulting taste more closely resembled that of sucrose.

The mixed compositions of glucose, fructose, and psicose prepared in Example 1 and Example 2 were also subjected to sensory testing, and as a result, they had a taste similar to that of sucrose.

### Example 3

### Preparation of acidic beverage containing D-fructose, D-glucose, and D-psicose

Beverages each containing the novel sweetener or sucrose were prepared according to the formulation shown in Table 2.

**[Table 2]**

| | Proportion | |
|---|---|---|
| D-fructose | 3.9 | |
| D-glucose | 4.8 | |
| D-psicose | 1.3 | |
| Sucrose | | 10 |
| Citric acid | 0.1 | 0.1 |
| Flavoring | 0.1 | 0.1 |
| Carbonic acid | 50 | 50 |
| Water | To make 100 | To make 100 |

As a result, both the obtained beverages had almost the same, pleasant degree and quality of sweetness.

### Example 4

### Physiologcal effects of sugar solution containing D-fructose, D-glucose, and D-psicose

In comparison with isomerized sugar of a known formulation, compositions each containing glucose, fructose, and psicose at a ratio of (1) 58:34:8 or (2) 60:35:5 were examined for their physiological effects using rats.

### Experimental Method (1)

Three-week-old male Wistar rats were used as experimental animals. The rats were divided into a psicose group (group given novel isomerized sugar) and a cellulose group (group given existing isomerized sugar) replacing psicose with cellulose. To the feed used for the experiment were added, in the case of the psicose group, glucose, fructose, and psicose at a ratio (weight) of 58:34:8 as carbohydrates; in the case of the cellulose group, the psicose was replaced with cellulose. The rats were fed ad libitum on the experimental feed and water for 5 weeks. After the feeding, the body fat was extracted, and the weight thereof was measured. Each measured value was expressed as a mean plus/minus standard error. Significance test was performed using the unpaired t-test.

### Results

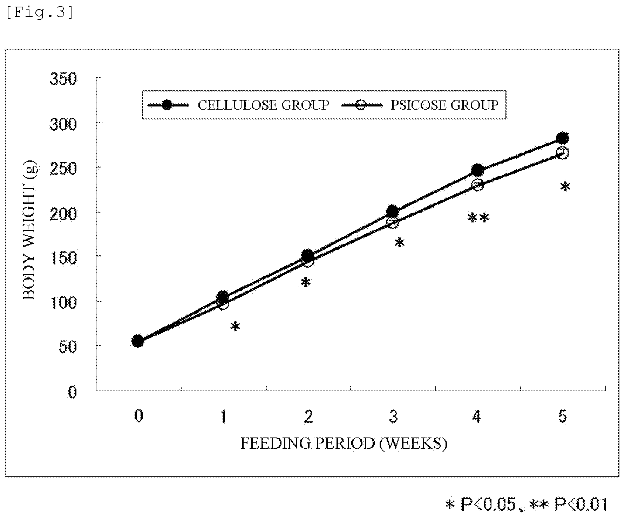

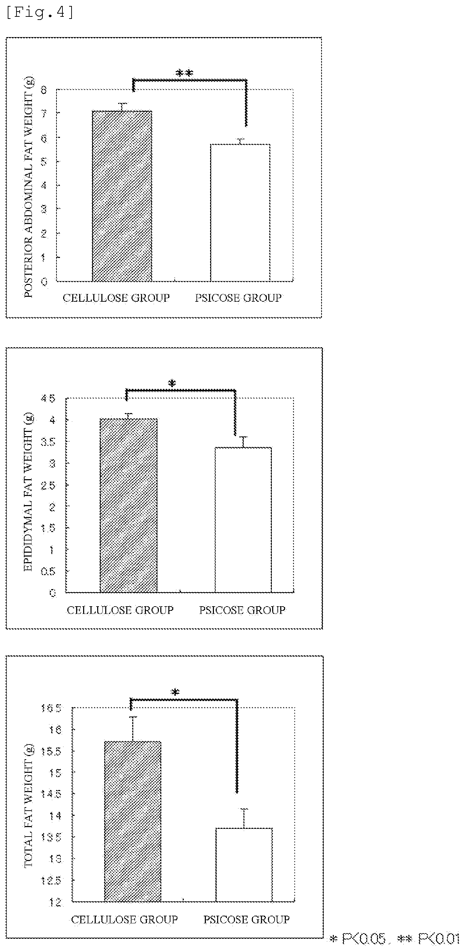

The average body weight of the Wistar rats at the start of the experiment was 55 ± 0.9 g (N = 18). As compared with the control group, a significant decrease in body fat weight was observed in the novel composition group (Tables 3 to 5 and FIGs. 3 to 5).

No difference was observed in feed intake between the psicose group and the cellulose group. (From observations during the experiment, no remarkable change was seen in the animals' behavior either.)

In the psicose group, the weight increase was suppressed than in the cellulose group, showing the effect of reducing overweight in animals induced by the intake of isomerized sugar.

**[Table 3]**

| Change in body weight | | | | | | |
|---|---|---|---|---|---|---|
| Age of rat | 4 weeks | 5 weeks | 6 weeks | 7 weeks | 8 weeks | 9 weeks |
| Testing period | Start | 1 week | 2 weeks | 3 weeks | 4 weeks | 5 weeks |
| Cellulose group | 55.7 ± 1.6 | 103.6 ± 2.5 | 150.6 ± 2.4 | 200.1 ± 4.3 | 245.9 ± 4.5 | 281.7 ± 5.6 |
| Psicose group | 55.3 ± 1.1 | 97.4 ± 1.1* | 144.1 ± 1.4* | 188.3 ± 2.0* | 229.5 ± 3.1** | 264.7 ± 4.5* |

| | | | | | | |
|---|---|---|---|---|---|---|
| Expressed as a mean ± standard error (g), *P < 0.05, **P < 0.01 | | | | | | |

**[Table 4]**

| | Fat tissue | | | |
|---|---|---|---|---|
| | Abdominal fat | Posterior abdominal fat | Epididymal fat | Total fat |
| Cellulose group | 4.6 ± 0.2 | 7.1 ± 0.3 | 4.0 ± 0.1 | 15.7 ± 0.6 |
| Psicose group | 4.6 ± 0.1 | 5.7 ± 0.2** | 3.4 ± 0.2* | 13.7 ± 0.5* |

| | | | | |
|---|---|---|---|---|
| Expressed as a mean ± standard error (g), *P < 0.05, **P < 0.01 | | | | |

**[Table 5]**

| Difference in ketone body | |
|---|---|
| | Amount of acetoacetic acid |
| Cellulose group | 126.0 ± 8.2 |
| Psicose group | 220.4 ± 26.4" |

| | |
|---|---|
| Expressed as a mean ± standard error (µmol/L), **P < 0.01 | |

### Experimental Method (2)

Three-week-old male Wistar rats were used as experimental animals. The rats were divided into a psicose group (novel isomerized sugar group) and a cellulose group (existing isomerized sugar group) where psicose is replaced with cellulose. To the feed used for the experiment were added, in the case of the psicose group, glucose, fructose, and psicose at a ratio (weight) of 60:30:5 as carbohydrates; in the case of the cellulose group, the psicose was replaced with cellulose. The rats were fed ad libitum on the experimental feed and water for 5 weeks. After the feeding, the body fat was extracted, and the weight thereof was measured. Each measured value was expressed as a mean plus/minus standard error. Significance test was performed using the unpaired t-test.

### Results

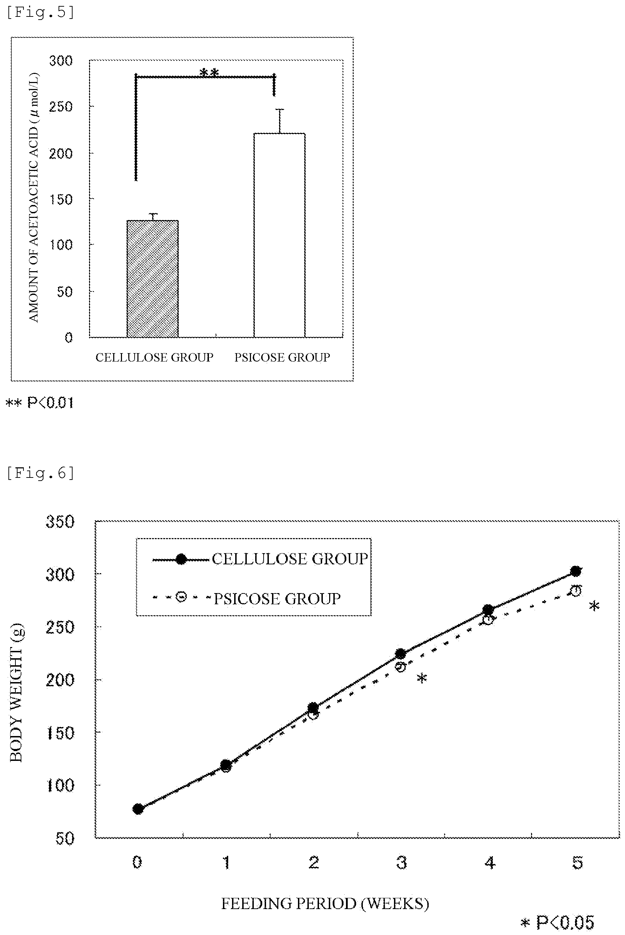

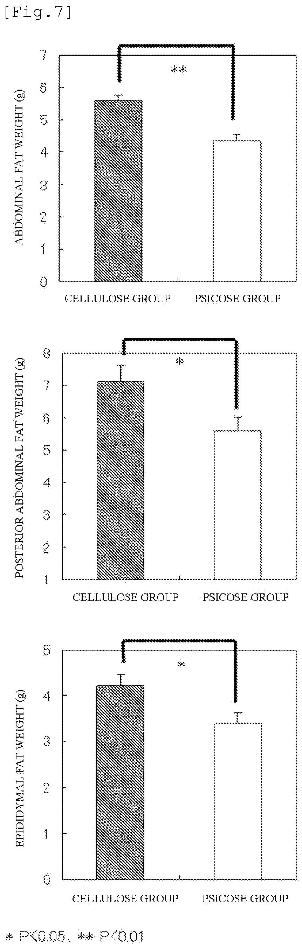

As compared with the cellulose (control) group, significant decreases in body weight and body fat weight were observed in the psicose group (Tables 6 to 8 and FIGs. 6 to 8) .

In the psicose group, the weight increase was suppressed than in the cellulose group, showing the effect of reducing overweight in animals induced by the intake of isomerized sugar.

**[Table 6]**

| Change in body weight | | | | | | |
|---|---|---|---|---|---|---|
| Age of rat | 4 weeks | 5 weeks | 6 weeks | 7 weeks | 8 weeks | 9 weeks |
| Testing period | Start | 1 week | 2 weeks | 3 weeks | 4 weeks | 5 weeks |
| Cellulose group | 76.8 ± 1.5 | 118.8 ± 2.0 | 173.1 ± 3.3 | 223.5 ± 4.1 | 265.4 ± 2.9 | 302.1 ± 3.5 |
| Psicose group | 77.1 ± 1.3 | 116.2 ± 1.5 | 166.9 ± 2.3 | 211.0 ± 3.5* | 255.9 ± 3.7 | 283.1 ± 5.7* |

| | | | | | | |
|---|---|---|---|---|---|---|
| Expressed as a mean ± standard error (g), *P < 0.05 | | | | | | |

**[Table 7]**

| | Fat tissue | | | |
|---|---|---|---|---|
| | Abdominal fat | Posterior abdominal fat | Epididymal fat | Total fat |
| Cellulose group | 5.6 ± 0.2 | 7.1 ± 0.5 | 4.2 ± 0.3 | 16.9 ± 0.9 |
| Psicose group | 4.3 ± 0.2** | 5.6 ± 0.4* | 3.4 ± 0.2* | 13.3 ± 0.8* |

| | | | | |
|---|---|---|---|---|
| Expressed as a mean ± standard error (g), *P < 0.05, **P < 0.01 | | | | |

**[Table 8]**

| Difference in ketone body | |
|---|---|
| | Amount of acetoacetic acid |
| Cellulose group | 159.3 ± 13.7 |
| Psicose group | 216.8 ± 11.2" |

| | |
|---|---|
| Expressed as a mean ± standard error (µmol/L), **P < 0.01 | |

### Discussion of ketone body

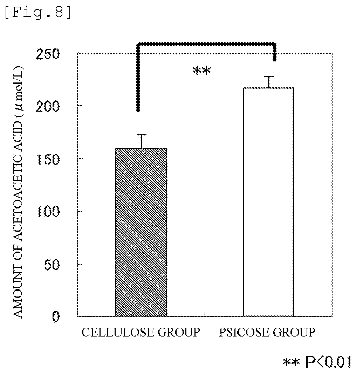

The above results show that D-psicose reduces body fat. Meanwhile, ketone bodies in blood plasma are known to be produced by fat burning in the liver. The D-psicose group exhibited a higher ketone body (acetoacetic acid) value than in the control group, and it is therefore estimated that fat burning in the liver was accelerated (FIG. 5, FIG. 8). Accordingly, the fat burning in the liver by D-psicose is thought to be one cause of the body fat reduction.

### Industrial Applicability

The present invention enables the provision of a novel sweetener that can be widely applied in the food industry or the like but never causes lifestyle-related diseases, such as obesity.

## Claims

1. A sweetener having the sweetness and taste of sucrose, which is a mixture of D-fructose, D-glucose, and D-psicose, for use in suppressing obesity induction by isomerized sugar,
wherein the amount of the D-fructose is 30 to 80 parts by weight, and the total amount of the D-glucose and the D-psicose is 70 to 20 parts by weight,
wherein the amount of the D-psicose is not less than 10 parts by weight based on 100 parts by weight of the total amount of the D-glucose and the D-psicose, and
wherein the sweetener contains the D-psicose in an amount of not less than 5 parts by weight.

2. The sweetener for the use according to claim 1, which is comprised in a food, a drink, a drug, a quasi drug, or a cosmetic product.

3. Non-therapeutic use of a sweetener having the sweetness and taste of sucrose, which is a mixture of D-fructose, D-glucose, and D-psicose, for suppressing an increase in body weight or an increase in body fat accumulation,
wherein the amount of the D-fructose is 30 to 80 parts by weight, and the total amount of the glucose and the psicose is 70 to 20 parts by weight
wherein the amount of the D-psicose is not less than 10 parts by weight based on 100 parts by weight of the total amount of the D-glucose and the D-psicose, and
wherein the sweetener contains the D-psicose in an amount of not less than 5 parts by weight.

4. The non-therapeutic use according to claim 3, wherein the sweetener is comprised in a food, a drink, a drug, a quasi drug, or a cosmetic product.

## Patentansprüche

1. Süßstoff mit der Süße und dem Geschmack von Saccharose, welcher eine Mischung aus D-Fructose, D-Glucose und D-Psicose ist, für die Verwendung in der Unterdrückung der Fettleibigkeitsinduktion durch isomerisierten Zucker,
wobei die Menge der D-Fructose 30 bis 80 Gewichtsteile und die Gesamtmenge der D-Glucose und der D-Psicose 70 bis 20 Gewichtsteile ist,
wobei die Menge der D-Psicose nicht weniger als 10 Gewichtsteile basierend auf 100 Gewichtsteilen der Gesamtmenge der D-Glucose und der D-Psicose ist, und
wobei der Süßstoff die D-Psicose in einer Menge von nicht weniger als 5 Gewichtsteilen enthält.

2. Süßstoff für die Verwendung nach Anspruch 1, welcher in einem Lebensmittel, einem Getränk, einem Arzneimittel, einem Quasi-Arzneimittel oder einem kosmetischen Produkt enthalten ist.

3. Nichttherapeutische Verwendung eines Süßstoffs mit der Süße und dem Geschmack von Saccharose, welcher eine Mischung aus D-Fructose, D-Glucose und D-Psicose ist, für die Unterdrückung eines Anstiegs im Körpergewicht oder eines Anstiegs in der Körperfettakkumulation,
wobei die Menge der D-Fructose 30 bis 80 Gewichtsteile und die Gesamtmenge der D-Glucose und der D-Psicose 70 bis 20 Gewichtsteile ist,
wobei die Menge der D-Psicose nicht weniger als 10 Gewichtsteile basierend auf 100 Gewichtsteilen der Gesamtmenge der D-Glucose und der D-Psicose ist, und
wobei der Süßstoff die D-Psicose in einer Menge von nicht weniger als 5 Gewichtsteilen enthält.

4. Nichttherapeutische Verwendung nach Anspruch 3, wobei der Süßstoff in einem Lebensmittel, einem Getränk, einem Arzneimittel, einem Quasi-Arzneimittel oder einem kosmetischen Produkt enthalten ist.

## Revendications

1. Edulcorant ayant la sucrosité et le goût du saccharose, qui est un mélange de D-fructose, de D-glucose et de D-psicose, pour une utilisation dans la suppression de l'induction de l'obésité par des sucres isomérisés,
dans lequel la quantité du D-fructose est de 30 à 80 parties en poids, et la quantité totale du D-glucose et du D-psicose est de 70 à 20 parties en poids,
dans lequel la quantité du D-psicose n'est pas inférieure à 10 parties en poids pour 100 parties en poids de la quantité totale du D-glucose et du D-psicose, et
dans lequel l'édulcorant contient le D-psicose en une quantité non inférieure à 5 parties en poids.

2. Edulcorant pour une utilisation selon la revendication 1, qui est incorporé dans un aliment, une boisson, un médicament, un quasi-médicament, ou un produit cosmétique.

3. Utilisation non thérapeutique d'un édulcorant ayant la sucrosité et le goût du saccharose, qui est un mélange de D-fructose, de D-glucose et de D-psicose, pour supprimer une augmentation de poids corporel ou une augmentation de l'accumulation de graisse corporelle,
dans laquelle la quantité du D-fructose est de 30 à 80 parties en poids, et la quantité totale du D-glucose et du D-psicose est de 70 à 20 parties en poids,
dans laquelle la quantité du D-psicose n'est pas inférieure à 10 parties en poids pour 100 parties en poids de la quantité totale du D-glucose et du D-psicose, et
dans laquelle l'édulcorant contient le D-psicose en une quantité non inférieure à 5 parties en poids.

4. Utilisation non thérapeutique selon la revendication 3, dans laquelle l'édulcorant est incorporé dans un aliment, une boisson, un médicament, un quasi-médicament, ou un produit cosmétique.
